# EUROPEAN PATENT SPECIFICATION

(11) **EP 0 443 231 B1**
(45) Date of publication and mention of the grant of the patent: **08.11.1995**
(21) Application number: 90301907.3
(22) Date of filing: 22.02.1990
(51) Int. Cl.: G01N 33/52, G01N 33/543, G01N 33/58

(54) **Multi-layered diagnostic test device for the determination of substances in liquids**
Mehrschicht-Testvorrichtung zur Bestimmung von Substanzen in Flüssigkeiten
Dispositif multicouche pour tests de diagnostic en vue de la détermination de substances dans des liquides

(43) Date of publication of application: 28.08.1991
(73) Proprietor: Editek, Inc., Burlington North Carolina 27215 (US)
(72) Inventor: Kuhn, Raymond E., Clemmons, NC 27012 (US); MacDonald, Gene H., Greensboro, NC 27408 (US)
(74) Representative: Perry, Robert Edward

(56) References cited:
- EP-A- 0 034 049
- EP-A- 0 066 648
- EP-A- 0 097 952
- EP-A- 0 345 781

## Description

This invention relates to a diagnostic test device for determining the presence of substances in liquid media. More specifically, it relates to a multi-layer test device for determining the presence of immunological, biological or enzymatic materials or other analytes in liquids, especially biological, industrial or agricultural liquids.

In the field of diagnostic testing, the art has evolved from the use of complex radioimmunoassays and enzyme immunoassays to the use of single card-type devices. In general, the art has sought to increase the readability of such devices, to eliminate the need for an instrument, and to render the device readable in a short period of time so that determination of the analyte may be made under field conditions.

A commonly-encountered problem in the field of immunoassay occurs when competitive or sandwich-type binding assays are performed on a solid porous matrix. The immunological reaction in such procedures is often neither sufficiently rapid nor conveniently visible in the absence of several wash steps or reagent additions. Even with the additional steps, the reaction is often not sufficiently visible within a convenient time to be useful.

In the typical competitive binding assay, an externally supplied tagged antigen competes with the antigen of a sample, to react with an externally-supplied antibody. (If the analyte is an antibody, the appropriate binding partners are chosen). Each antigen molecule, whether tagged or present in the sample, has an opportunity to react with the supplied antibody and the extent to which it does react is a measure of the concentration of the antigen in the original sample.

Theoretically, it is possible to perform a competitive assay by displacing tagged antigen from a tagged antigen/antibody complex by contacting the complex with antigen from the sample. Thus, if a tagged antigen/antibody complex is contacted with a source of untagged antigen, one might expect a displacement of the tagged antigen to occur. Unfortunately, when the solid substrate is a porous material, the mere contact does not always result in a displacement which is suitable for commercial analyte detection.

According to the present invention, a composite device, suitable for use in detecting or determining the presence of components in liquids, comprises first and second members having facing surfaces in contact, the members being each of porous material and having different pore sizes; a sample-receiving site; a reaction site in communication with the sample-receiving site via the second member; and, located in the members, liquid barrier means defining a liquid flow path, whereby liquid deposited at the sample-receiving site is transferred to the reaction site through both said members.

A device of the present invention is portable, can develop a visual colour change almost immediately upon the introduction of test sample, and is readable by the naked eye in a remote location without the need for instrumentation. Its use is convenient, since it can eliminate multiple wash steps and long incubation times, so that the time for reading the test is relatively short. The device can be employed at the site and in the field and at the immediate location where substances are to be detected. Its use requires little or no further intervention prior to the development of the reaction for the visualisation of the end result.

In the novel composite structure of porous materials, the selection of different pore sizes in appropriate areas of the structure, and the juxtaposition of liquid barrier means, defines a liquid flow path which results in an unexpected liquid flow force sufficient to cause a discernible change in an immunological complex or in other reactions when appropriate materials are selected. More specifically, use of the novel device facilitates, in a simple one-step operation, the displacement of tagged antigen from a tagged antigen/antibody complex by sample antigen (if the complex is originally configured and supplied in this manner) or the effective competition between the sample analyte and externally supplied tagged analyte for the immobilised capture binding partner, with rapid revelation of a visual indication of such reaction.

Other forms of immunoassay formats such as sandwich reactions and competitive binding using a detection system for antibodies as the analyte rather than antigens as described, may also be employed as will be seen in the detailed description of the invention.

Briefly, and without regard to the drawings at this point, a structure is provided with a combination of various pore sizes and liquid flow barriers so as to produce rapid wicking of a sample in a pre-determined liquid flow direction to a reaction site at which the desired reaction takes place.

In its most generalized form, the present invention contemplates a structure having at least two planar, flat porous members in intimate contact with each other at their planar surfaces. The multi-layered device is configured in such a way that when a small amount, such as a drop or two, of a liquid test sample is placed onto or into a sample receiving site provided at the top member, the liquid is forced to travel transversely into the bottom member owing to the presence of liquid flow barriers placed or created adjacent to the sample receiving site. The sample receiving site may be a hole in the top member, or may be the top member itself appropriately shaped or confined in consideration of the type of test to be performed.

The device is configured in such a way as to force the flow of sample liquid from the sample receiving port or site along a pre-determined pathway to the bottom member and ultimately to a reaction site on the bottom or top member depending upon the pre-determined path selected. The sample liquid thus ultimately reaches the reaction site not by lateral flow from the sample receiving site, but rather by traversing the sample receiving site in or on the top member in a transverse flow into the second member and up into the top member. Usually, the reaction site is placed on the top member. It may be placed at the bottom member under appropriate circumstances.

The characteristics of the members can be varied in such a way as to create either a rapid pulling action from the top member and/or a pumping action due to differential wicking characteristics between the lower member and the top member. Sample flow in the second member which receives the sample from the sample receiving site is also restricted by barrier means which constrain the flow of liquid in the second member to a defined space and a direction of that flow up into the first member.

By this action there is facilitated a wide variety of potential reactions and reaction sites. For example, the analyte of the sample dropped onto a top member in a manner in which the sample is prevented from flowing laterally to any substantial degree across the top member but is constrained to flow transversely, can be ultimately transferred to a reaction site located on the top member. This reaction site (whether located on the top member or on the bottom member) may either have reagents deposited thereon or may be itself a receiving site for additional reagents either directly applied or directed through the same route as the original sample deposition. The present invention requires the placement of a defined sample receiving site juxtaposed with certain liquid flow barriers and a reaction site in a pre-determined fashion so as to direct the flow of the sample liquid in the pre-selected fashion.

The versatility of the device of the present invention is quite wide. For example, the members themselves may be either hydrophilic or hydrophobic depending on the test characteristics and the analytes to be tested. For example, by appropriate choice of structure and composition of members, analyses of aqueous or non-aqueous samples may be performed. Non-aqueous samples containing organic solvent-based material may be employed with hydrophobic members in appropriate circumstances. The invention also permits the use of hydrophobic material with aqueous samples should personal preferences dictate. Examples of hydrophobic materials are glass fiber, certain nylons, teflon, and polyvinyl chloride polymers. Examples of hydrophilic materials are paper, certain cellulose acetates, polyvinylidene difluoride, cellulose nitrate, polypropylene, certain microfiberglass compositions and the like.

A combination of members with different porosities and binding characteristics with the added capability of being either hydrophilic or hydrophobic provides in the device a) the ability to perform on both aqueous and nonaqueous samples, b) the binding of reactants on one or both of the members and at different sites in the flow path of sample or reagents, and c) through impermeable barriers or slots, the ability to differentially control flow rates.

In one form of an immunoassay, the sample is added to the sample port to hydrate a reactant in the bottom or lower member. The reactant may be a competing analyte bound to an indicator such as enzyme or colloidal gold. A binding (capture) antibody is in this case, located at the reaction site. The member may be protein-binding, if desired to retain the capture antibody. The lower member could be non-protein-binding in the region of the placement of the indicator conjugate but could be protein-binding at the terminus past the reaction site to retain reactants and prevent them from diffusing back to the reaction site.

In another assay, the bottom member could be initially protein binding and during manufacture have adhered thereto one or more enzymes for an assay to determine the presence of a substrate in a sample solution. The sample would move through the lower member contacting specific enzymes in a determined sequential manner to effect the production of a product which could then be observed at the reaction site.

It will be obvious to those skilled in the art that numerous modifications of immunoassay procedures can be performed on the device. These include but are not limited to competitive assays for small analytes, sandwich assays, and direct detection of reactants in samples. It will also be obvious that other non-immunologically based assays, such as substrate and product detection, use of nucleic acid probes, lectins, or any other ligand-receptor pairs with various indicator systems can be performed on the device as well.

To illustrate further, when a liquid sample is applied to the sample site in or on the upper member, the liquid will wick by capillary action into the lower member in either a rapid fashion or slower fashion depending upon the characteristics of the member selected. It is preferred under certain circumstances that the lower member have a pore size substantially larger than the pore size of the upper member to facilitate a pumping action on the return flow from the bottom member to the top member. Sample is prevented from moving laterally from the sample receiving site because the edge of the sample site has been rendered impermeable by the installed barrier means. These barrier means may be compression sites, slots, discontinuities in the material, sonic or heat-generated barriers and the like and are all within the skill of the art to construct and place.

Once liquid enters the lower member it moves laterally and transversely until it contacts an impermeable barrier installed in the lower member and juxtaposed in such a way as to permit flow of liquid back up into the top member therein to come in contact with reagents or the like at a reaction site. The reaction site may contain the elements of an indicator system useful in detecting the analyte (or substrate) of interest. In addition, if desired, the reaction site may be located in the lower member with the top member serving as the remote site for accumulation of reactants and sample past the reaction site.

For example, in a preferred embodiment, wherein a competitive immunoassay is performed, the reaction site may conveniently contain antibodies (capture antibodies) to the analyte of interest, which said antibodies are covalently bonded or otherwise attached to the upper member. In this regard, if desired, a protein-binding type of member may be selected as the top member to facilitate the binding of the antibody. A conjugate of an indicator molecule attached to the analyte of interest is selected. A preferred conjugate is the analyte bound directly to colloidal gold if feasible, or to a carrier molecule if, for example, the analyte is unable or poorly able to bind to the gold itself. Colloidal gold is a well-known reagent used in diagnostic procedures because of its characteristic reddish color. As carrier molecules there may be employed for example, natural or synthetic proteins or other macromolecules such as BSA, poly L-Lysine, polysaccharides, histones, casein, horseradish peroxidase and the like. The conjugate may be admixed with the sample prior to applying the sample to the receiving site or may be installed in the device somewhere in the pre-determined liquid flow path prior to the reaction site. If the analyte in the sample is homologous to the analyte adhered to the colloidal gold, it will compete with the analyte-gold conjugate at the reaction site for the capture antibody. Assuming appropriate selection of antigen/analyte-gold conjugate concentration in consideration of the conditions of the assay, the analyte-gold conjugate will lose in the competition to the analyte in the sample, and no conjugate will remain bound to the capture antibody. The reaction can then be traced by the absence of the accumulation of gold at the reaction site. Thus, a positive reaction is signified by a lack of change of color at the reaction site (i.e., absence of conjugate). This reaction, normally termed a competitive binding assay, is typical of the ones that may be performed with the device of the present invention. Other formats may be used as well as will be described hereinafter.

The following is a brief description of the drawings presented herein from a consideration of which the present invention will be further understood.

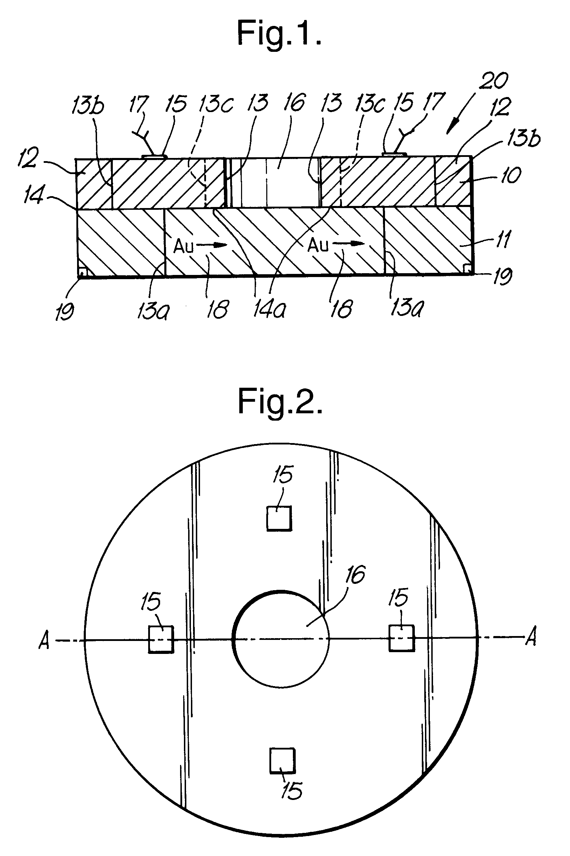

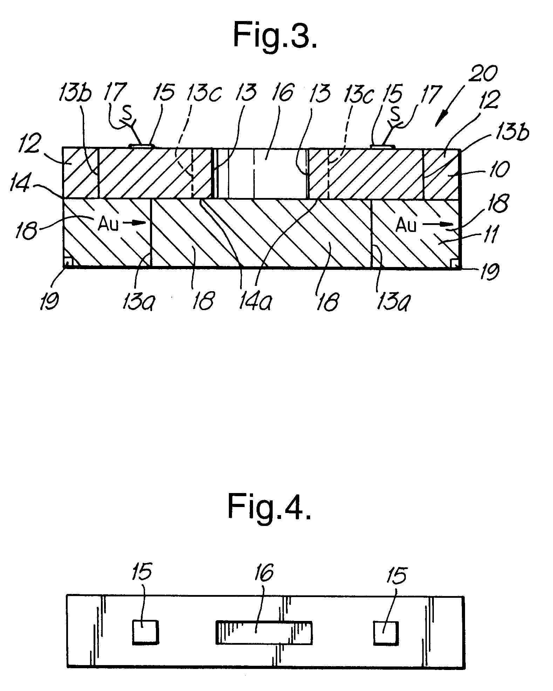

Figure 1 is a cross-sectional view taken across the plane A-A of Figure 2 of a device of the present invention. Figure 2 is an embodiment of the device of the invention shown in circular form. Figure 3 is a device of the invention shown after it has been acted upon by a test liquid operating on the device of Figure 1. Figure 4 is a top view of another embodiment of a device of the present invention.

Figure 1 shows a bilayer device 10 and two of the four reaction sites 15 that are shown in Figure 2. An upper member 20 is provided with a sample port 16 bounded by barriers 13. A lower member 11 has vent ports 19. The members 11,20 communicate at interface 14. The members 11,20 are each composed of one layer. They may be the same or different material and have different porosities. In a preferred embodiment of the invention, the bottom member has a pore size about 5 to 10 times greater than the pore size of the upper member, and the pore size of the upper member is in the range of 0.2-0.75 microns.

With regard to the varying pore sizes, it should be noted that the larger pore size should not be so large as to provide a sink for the liquid, since this will frustrate the pumping action desired. What is highly desirable, however, is a pore size that results in a "push" of the sample through the sample port into the lower member followed by a "pull" (as will be described later) of the liquid from the lower member back into the first member.

Members 11 and 20 are also equipped with barriers 13a and 13b which are generally incorporated during the manufacturing process as by welding or by incorporation of slots, discontinuities or the like. Barriers 13b are incorporated to provide additional reservoir compartments and are optional depending on the size of the reservoir desired. Barriers 13c are also optional; in practice, the barrier at 13 is usually sufficient. There may be some circumstances, however, where the kinetics of the test and the device size are such that it would be desirable to direct the upward flow of liquid into the top member at a point somewhat remote from the sample receiving site. In such a case, barriers 13c may be provided and interface 14a rendered impermeable or not, as desired.

Present on member 20 are reaction sites 15 containing various reactants placed in accordance with and in consideration of the ultimate test that is to be performed. As shown in Figure 2, these reaction sites may be more than one in number and may be for the same or different analytes coming from the same sample or may be for a control. The device 10 may also have either antibodies or antigens, but preferably in the discussion given herein, will have antibody 17 attached to the member 10 either by covalent bonding or some other physical or chemical attachment. There is also provided in member 11, at 18, a conjugate shown as Au → of colloidal gold or some other indicator system conjugated to an antigen which is specific for or will react with the antibody at 17. Thus, in its completed composite form ready for use, there is a conjugate 18 of the gold (or other indicator system) to the antigen incorporated into the flow path of the device prior to the reaction site 15. Alternatively, the conjugate may be admixed with sample instead of being incorporated into the device, and the sample/conjugate mixture be allowed to follow the flow path to reaction site 15.

Instead of gold, there may be employed any other detection systems used in immunoassays such as enzymes, fluorescing agents, latex beads, luciferases, chemilumniscent agents and the like depending upon the best mode of reaction for the given analyte as determined by individual preferences.

In use, a liquid sample is dropped into sample port 16 and the sample allowed to diffuse into member 11 and mix with conjugate 18. Lateral flow is prevented by the barriers 13 which direct flow of the sample into the second member. Upon reaching the second member, the liquid is directed laterally until it reaches a barrier (e.g. 13a) or until it is not permitted to go any further and is "pulled" into member 20 in admixture with conjugate 18. The mixture then migrates to the reaction site 15. At the reaction site 15, if the sample contains analyte corresponding to the antigen which is conjugated to the indicator system, it will compete with the conjugate in reaction with antibody 17. If the sample contains no such antigen, then the only reactant at site 15 other than antibody 17 will be conjugate which will then react and show a color change. If sample does contain relevant antigen, the reaction at 15 will be almost exclusively due to the presence of such antigen because the concentration of conjugate and antibody 17 selected in constructing the device has deliberately been adjusted to favor reaction from the normally encountered concentration of analyte in the sample. Thus, a positive result is shown as no color change. For example, in Figure 3 such a result is shown by an "S" for sample being attached to the antibody 17. The gold/antigen complex does not attach at reaction site 15 and is further removed from that area by the flow of liquid continuing past the reaction site and into the further reaches of members 20 and 11. If desired, larger amounts of reservoir type material can be supplied simply by extending member 20 laterally or providing additional reservoir space in member 11 depending upon how much reservoir space is needed as a function of the test that is being performed. Also, a reservoir of absorbent material may be placed in juxtaposition with member 20.

Following addition of the sample, it is desirable though not necessary in many cases, to add a wash solution to further direct the sample away from the reaction site so that any indicator antigen complex adjacent to the reaction site is moved further away as is evidenced by Figure 3.

If the reaction device as shown in Figure 4 for example, is equipped with viewing ports so that the migration of the gold or indicator system away from the reaction site 15 is obscured, then a change in color at the reaction site 15 is all that one needs to observe. Sufficient wash material or sample may be applied to permit the visualization of the clear spot in the event that sample does contain the suspected analyte.

Although Figure 2 has been shown as being circular in form, it may be of any convenient shape such as rectangular, cross-shaped or the like. In addition, it need not be restricted to one reaction site or sample receiving port but may include a variety and a plurality of either or both of those and may include reactants for detection of various analytes.

With respect to the material that may be employed as the members, particular success has been observed with polyvinylidene difluoride with pore sizes of about 0.2 to 0.75 microns for the top member and from 3-5 microns for the lower member. Various other materials may be employed such as cellulose acetates, cellulose nitrates, polypropylenes, certain microglass fiber compositions and the like.

Although the above description has been given with reference to a competitive binding assay wherein the analyte to be detected is an antigen and the immunoreagent at reaction site 15 is an antibody and the indicator system at 18 is gold complexed to an antigen which binds with the antibody, the present invention is also suitable for a competitive assay in which the analyte is an antibody to be determined instead of an antigen. Moreover, the present invention is also suitable for the detection of an antigen or antibody as an analyte wherein a sandwich technique is employed for the reaction. For example, in an immunoassay for human chorionic gonadotropin (hCG), gold conjugated to anti-beta hCG is placed in the bottom member at 18 (Figure 1) and anti-alpha hCG (capture Ab) is covalently or otherwise attached to the upper member at reaction site 15. If sample contains hCG it binds to the gold/antibody conjugate and migrates to site 15 where the sample hCG part of the complex binds to the capture antibody (17) and yields a red color at site 15 indicating a positive result. If hCG is absent from the sample, the gold-anti-beta hCG complex has no hCG bound to it. The complex would not bind to capture antibody 17 but would instead migrate past the reaction site to the remote reaches of member 20 and/or member 11 (e.g. to 12 on member 20).

## Claims

1. A composite device suitable for use in detecting or determining the presence of components in liquids, which comprises first and second members (11,20) having facing surfaces in contact, the members being each of porous material and having different pore sizes; a sample-receiving site (16); a reaction site (15) in communication with the sample-receiving site via the second member; and, located in the members, liquid barrier means (13) defining a liquid flow path, whereby liquid deposited at the sample-receiving site is transferred to the reaction site through both said members.

2. The device of claim 1, wherein the first and second members are each flat and planar.

3. The device of claim 1 or claim 2, wherein the porous material is selected from polyamide, polytetrafluoroethylene, glass fibres, polyvinyl chloride, cellulose acetate, polyvinylidene difluoride, cellulose nitrate and polypropylene.

4. The device of any preceding claim, wherein the sample-receiving site is on the first member, and the reaction site is on the first or second member.

5. The device of any preceding claim, for the determination of one or more immunological components of a liquid, wherein one or more binding partners (17) of the immunological component(s) to be determined is or are present at the reaction site or a plurality thereof.

6. The device of claim 5, wherein the binding partner is an antibody and the component to be determined is an antigen.

7. The device of claim 6, wherein the antibody or an antigen corresponding to the component to be determined is present in the second member, in the liquid flow path prior to the reaction site, the antibody or antigen being conjugated to an indicator system.

8. The device of claim 7, wherein the indicator system is colloidal gold.

9. The device of any preceding claim, wherein the barrier means (13) comprises slots, discontinuities, welds or compressions.

10. The device of any preceding claim, which comprises reactants disposed in the liquid flow path, prior to the reaction site, such that, when sufficient liquid flows along the path, the reactants are delivered to the reaction site.

## Patentansprüche

1. Verbundeinrichtung, geeignet zur Verwendung zum Bestimmen oder Feststellen der Gegenwart von Komponenten in Flüssigkeiten, die folgendes umfaßt:
ein erstes und zweites Glied (11,20), deren gegenüberliegende Flächen in Kontakt stehen, wobei die Glieder jeweils aus porösem Material sind und unterschiedliche Porengrößen besitzen; eine probenaufnehmende Stelle (16); eine Reaktionsstelle (15), die mit der probenaufnehmenden stelle über das zweite Glied in Kommunikation steht; und in den Gliedern angeordnete Flüssigkeitsbegrenzungsmittel (13), die den strömungsweg der Flüssigkeit begrenzen, wodurch die an der probenaufnehmenden Stelle abgegebene Flüssigkeit durch beide Glieder zur Reaktionsstelle überführt wird.

2. Einrichtung nach Anspruch 1, wobei sowohl das erste als auch das zweite Glied jeweils eben und planar ist.

3. Einrichtung nach Anspruch 1 oder 2, wobei das poröse Material aus Polyamid, Polytetrafluorethylen, Glasfasern, Polyvinylchlorid, Celluloseacetat, Polyvinylidendifluorid, Cellulosenitrat und Polypropylen ausgewählt wird.

4. Einrichtung nach irgendeinem vorstehenden Anspruch, wobei die probenaufnehmende Stelle am ersten Glied ist, und die Reaktionsstelle am ersten oder zweiten Glied vorgesehen ist.

5. Einrichtung nach irgendeinem vorstehenden Anspruch, zur Bestimmung einer oder mehrerer immunologischer Komponenten einer Flüssigkeit, wobei ein oder mehrere Bindungspartner (17) der zu bestimmenden immunologischen Komponente(n) an der Reaktionsstelle oder einer vielzahl davon, vorhanden ist oder sind.

6. Einrichtung nach Anspruch 5, wobei der Bindungspartner ein Antikörper und die zu bestimmende Komponente ein Antigen ist.

7. Einrichtung nach Anspruch 6, wobei der Antikörper oder ein Antigen, das der zustimmenden Komponente entspricht, im zweiten Glied, im Strömungsweg der Flüssigkeit vor der Reaktionsstelle, vorhanden ist, und wobei der Antikörper oder das Antigen mit einem Anzeigesystem konjugiert ist.

8. Einrichtung nach Anspruch 7, bei der das Anzeigesystem kolloidales Gold ist.

9. Einrichtung nach irgendeinem vorstehenden Anspruch, bei der das Begrenzungsmittel (13) Schlitze, Diskontinuitäten, Schweißungen oder Zusammendrückungen umfaßt.

10. Einrichtung nach irgendeinem vorstehenden Anspruch, die Reaktanten umfaßt, welche im Strömungsweg der Flüssigkeit vor der Reaktionsstelle derart vorliegen, daß wenn ausreichend Flüssigkeit den Weg entlangströmt, die Reaktanten zur Reaktionsstelle geliefert werden.

## Revendications

1. Dispositif composite convenant pour une utilisation dans la détection ou la détermination de la présence de composants dans des liquides, qui comprend un premier et un second éléments (11, 20) présentant des surfaces opposées en contact, les éléments étant chacun constitués d'un matériau poreux et présentant des tailles de pores différentes; un site (16) de réception d'échantillon; un site de réaction (15) en communication avec le site de réception d'échantillon par l'intermédiaire du second élément; et, situés dans les éléments, un moyen (13) de barrière au liquide définissant un parcours d'écoulement pour le liquide, grâce auquel un liquide déposé dans le site de réception d'échantillon est transféré vers le site de réaction à travers les deux éléments.

2. Dispositif selon la revendication 1, dans lequel le premier et le second éléments sont chacun plans et unis.

3. Dispositif selon la revendication 1 ou la revendication 2, dans lequel le matériau poreux est choisi parmi le polyamide, le polytétrafluoroéthylène, des fibres de verre, le chlorure de polyvinyle, l'acétate de cellulose, le difluorure de polyvinylidène, le nitrate de cellulose et le polypropylène.

4. Dispositif selon l'une quelconque des revendications précédentes, dans lequel le site de reception d'échantillon est situe sur le premier élément, et le site de réaction est situé sur le premier ou le second élément.

5. Dispositif selon l'une quelconque des revendications précédentes, pour la détermination d'un ou de plusieurs composants immunologiques dans un liquide, dans lequel un ou plusieurs partenaires de liaison (17) du ou des composants immunologiques à déterminer est ou sont situes sur le site de réaction ou sur plusieurs de ceux-ci.

6. Dispositif selon la revendication 5, dans lequel le partenaire de liaison est un anticorps, et le composant à déterminer est un antigène.

7. Dispositif selon la revendication 6, dans lequel l'anticorps ou un antigène correspondant au composant à déterminer est situé dans le second élément, dans le parcours d'écoulement du liquide avant le site de réaction, l'anticorps ou l'antigène étant conjugué à un système d'indicateur.

8. Dispositif selon la revendication 7, dans lequel le système d'indicateur est de l'or colloïdal.

9. Dispositif selon l'une quelconque des revendications précédentes, dans lequel le moyen de barrière (13) comporte des fentes, des discontinuités, des soudures ou des compressions.

10. Dispositif selon l'une quelconque des revendications précédentes, qui comporte des réactifs disposés dans le parcours d'écoulement du liquide avant le site de réaction de telle sorte que, lorsqu'une quantité suffisante de liquide s'écoule le long du parcours, les réactifs sont délivrés au site de réaction.
